Europäisches Patentamt

European Patent Office

Office européen des brevets

(19)

(11) **EP 1 493 382 A2**

(12) **EUROPEAN PATENT APPLICATION**

(43) Date of publication:
05.01.2005 Bulletin 2005/01

(51) Int Cl.7: **A61B 5/0275**, A61B 8/00

(21) Application number: 04076913.5

(22) Date of filing: 02.07.2004

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK** | (72) Inventors:<br>• **Korsten, Hendrikus Hubertus Maria**<br>  **5615 EG Eindhoven (NL)**<br>• **Mischi, Massimo**<br>  **5632 HC Eindhoven (NL)** |
| (30) Priority: **03.07.2003 EP 03077095** | (74) Representative:<br>**Dohmen, Johannes Maria Gerardus et al**<br>**Algemeen Octrooi- en Merkenbureau**<br>**P.O. Box 645**<br>**5600 AP Eindhoven (NL)** |
| (71) Applicant: **TECHNISCHE UNIVERSITEIT EINDHOVEN**<br>**5612 AZ Eindhoven (NL)** | |

(54) **Method and arrangements for determining an indicator dilution curve (IDC) of an indicator in a bloodstream of a human or animal body**

(57)     A method and an arrangement (50-53) for determining cardiac parameters of a human or animal body (60) having a central bloodstream comprising an indicator periodically passing one or more locations (1) of the body (60). An ultrasound signal is applied (53) to the indicator in the central bloodstream and ultrasound measurements (53) are taken from a return signal in response to the ultrasound signal, of at least a first cycle of the indicator. From the ultrasound measurements (53) an indicator concentration time signal is determined. The indicator is of such a low concentration that there is a descriptive relation between the measurements and the indicator concentration time signal. From the first cycle of the indicator concentration time signal an indicator dilution curve is modelled (50) and the modelled indicator dilution curve is adjusted over a time interval. The time interval corresponds to at least part of the first cycle leading a further cycle of the indicator. Cardiac parameters are determined (50) from a plurality of modelled indicator dilution curves obtained from a plurality of locations in the central bloodstream of the body (60).

FIG. 6

EP 1 493 382 A2

**Description**

<u>Field of the Invention</u>

The present invention relates to a method and an arrangement for determining cardiac parameters from a time-dependent concentration of an indicator passing one or more locations in a bloodstream in a human or animal body based on ultrasonic measurements.

<u>Background of the Invention</u>

The measurement of cardiac output (C0) is common practice in cardiology on the coronary care unit, or in the operating room during heart surgery, or in the intensive care in order to monitor the condition of a patient. The cardiac output may be defined as the volume of blood per unit of time ejected by the left ventricle of the heart into the aorta, or ejected by the right ventricle of the heart into the pulmonary artery. Since the bloodstream of a human or animal body is a closed flow system, conservation of mass teaches that the cardiac output is independent to where in the bloodstream it is measured.

Other physiologic parameters that are used to determine a persons condition or health are the ejection fraction (EF), the regurgitant fraction, and the intra-thoracic blood volume. The ejection fraction is a parameter that is considered of great importance for heart performance evaluation, and represents a non-dimensional estimation of the efficiency of the heart. The ejection fraction of the left or right ventricle equals the blood volume which is ejected by the left or right ventricle each heartbeat (referred to as stroke volume (S), i.e. the difference between the end-diastolic and the end-systolic volume of the left or right ventricle), divided by the end-diastolic volume ($V_{ed}$) of the left or right ventricle.

The ejection fraction can be separated into two components: the forward ejection fraction which refers to the blood volume that is ejected by the ventricle into the artery, and the regurgitant ejection fraction which refers to the blood volume that is ejected backward into the atrium due to valve insufficiency.

The intra-thoracic blood volume is the amount of blood present between the right ventricle and the left atrium, and is indicative of the amount of blood in pulmonary circulation.

These and other physiologic parameters may be derived from measurements of concentrations of a for instance a contrast agent that was brought into the bloodstream.

Methods that are used for measuring the cardiac output are based on injections of amounts of a tracer or an indicator in the bloodstream and measuring the indicator concentration over time in one or more regions of interest. This may be performed, for instance, by inserting one or more catheters, as will be described below. By evaluating gradual change of indicator concentration as the indicator passes a certain location or region of interest for the first time, it is possible to calculate physiologic parameters such as cardiac output and ejection fraction.

When an indicator bolus passes a certain location, the indicator concentration increases rapidly at first as the bulk of the indicator bolus passes, after which the indicator concentration gradually decreases again as the tail of the bolus passes. The tail of the concentration curve (indicator concentration versus time) is important, as most of the information about the flow and ejection fraction may be achieved by measuring the characteristics of the tail of the indicator concentration as it passes along the location for the first time.

Measurement of the intra-thoracic blood volume requires insertion of a measurement catheter through the femoral artery up to the aorta, close to the left ventricle, and an additional injection catheter through the right side of the heart (through the right atrium and ventricle) up to the pulmonary artery. The employment of two catheters allows measurement of the intra-thoracic mean transit time (MTT), which is the average time that the contrast takes to cover the pulmonary circulation distance. A major disadvantage of this technique is the fact that it is highly invasive and cannot be easily carried out in emergency circumstances, or on an "out-patient" basis.

Some non-invasive techniques are based on imaging techniques, such as ultrasound imaging. A major disadvantage of using these imaging techniques is that the relation between the measurements that are taken is blurred by a number of non-ideal 1 effects, such as attenuation for ultrasound imaging, for example, which is related to the presence of the indicator in the region of measurement. Due to this, it becomes extremely difficult to find a suitable relation between the ultrasound measurements and the concentration in any given case; the relation becomes to some extend unpredictable and the measured concentration values may be questionable. Accordingly, it is uncertain whether ultrasound measurement techniques are suitable for medical purposes. It is cumbersome to find the concentration values, and the consequences of making a wrong diagnosis based on physiological parameters that are based on erroneous concentration values may be fatal for the patient involved.

<u>Summary of the Invention</u>

It is an object of the present invention to provide a reliable method for determining cardiac parameters, using ultrasound imaging techniques.

These and other objects are achieved in accordance with the present invention by providing a method for determining cardiac parameters of a human or animal body having a central bloodstream comprising an indicator periodically passing one or more locations of the body, the method comprising the steps of:

applying an ultrasound signal to the indicator in the central bloodstream;

taking ultrasound measurements from a return signal in response to the ultrasound signal, of at least a first cycle of the indicator;

determining an indicator concentration time signal from the ultrasound measurements, the indicator being of such a low concentration that there is a descriptive relation between the measurements and the indicator concentration time signal;

modelling an indicator dilution curve from the first cycle of the indicator concentration time signal and adjusting the modelled indicator dilution curve over a time interval, wherein the time interval corresponds to at least part of the first cycle leading a further cycle of the indicator;

wherein the cardiac parameters are determined from a plurality of modelled indicator dilution curves obtained from a plurality of locations in the central bloodstream.

For ultrasound measurement techniques, as described above, it has surprisingly been observed that for measurements of concentrations of an indicator which are substantially less compared to the regular concentrations used in ultrasound imaging techniques according to the prior art, the relation between the measurements and the indicator concentration becomes descriptive.

In an embodiment of the invention, the descriptive relation is a mathematical relation, such as a logarithmic relation or a linear relation.

This may be explained by the fact that the effect of attenuation in ultrasound measurement techniques can be neglected for very small doses of indicator in the bloodstream. The indicator concentrations that are detected in an embodiment according to the invention are many orders of a magnitude smaller than the regular concentrations used.

For example, where in regular ultrasound measurement techniques a dose of 5 ml indicator (such as ultrasound contrast agent) may be required for detection in the bloodstream of an adult person, in an embodiment according to the invention measurements with a 100 times diluted dose of a 5 ml indicator present in the bloodstream are performed. Such a dose may be termed "ultra low" compared to the prior art.

Since the relation becomes descriptive for such small concentrations of indicator, this makes the method according to the invention reliable and relatively easy and efficient, enabling the use of equipment with limited arithmetic capabilities or processing power, that still provide sufficient performance to carry out the method accurately and very quickly.

Using an ultra low dose of indicator in the bloodstream as suggested above has another important benefit, in that the method can be carried out by a technician or any other person, without the requirement for a medical training or even automatically, without supervision by a physician or another medically trained person.

Another important benefit of the method according to the present invention is that, due to the ultra low doses of indicator used, an indicator bolus used for a measurement dissolves after a few number of blood cycles. Therefore, a new measurement can be performed shortly after an earlier measurement has been taken, and new measurements are not disturbed or blurred by traces of indicator present in the bloodstream as a result of earlier measurements.

According to a preferred embodiment of the present invention, the measurements are performed at a plurality of the locations simultaneously.

A benefit of this embodiment is that indicator concentrations may be monitored/detected at various locations in the bloodstream over a certain period of time, such that parameters as mean transit time may be determined.

With the help of any of the embodiments described here, a number of physiological parameters may be determined accurately. Therefore other embodiments of the invention are directed to determining the volumetric flow of blood in a bloodstream, the ejection fraction of any of the ventricles of the heart, the regurgitation fraction of any of the ventricles of the heart, the total blood volume by determining and multiplying cardiac output and recirculation mean transit time, and the intra-thoracic blood volume by determining and multiplying the cardiac output and the intra-thoracic mean transit time (MTT) in pulmonary circulation. Moreover the ratio between the intra-thoracic blood volume and the total blood volume may be assessed by comparison of the intra-thoracic mean transit time with the recirculation mean transit time.

The measurements of multiple dilution curves, measured simultaneously at different sites in the central bloodstream circulation, can also pass through a deconvolution technique, which allows estimating the dilution impulse response of the fluid-dynamic system between two different locations. Still two curves, one for each location, are needed in order to apply the deconvolution technique and estimate the impulse response. This approach can be used for several measurements, such as the pulmonary blood volume or the ventricular ejection fraction.

Since the bloodstream is a closed flow system it will be apparent that some time after a first passage of an indicator, recirculation of the indicator will take place and the tail of the first passage will be overtaken by the head of the second passage. Measuring the indicator concentration in the tail of the first passage of the indicator at a certain location is therefore troubled by the effects of recirculation.

However, by adjusting, in accordance with an embodiment of the invention, a modelled indicator dilution curve (IDC) over a time interval corresponding to a part of the first cycle leading a second cycle of the indicator periodically passing a location, while measurements outside that interval may be ignored, it becomes possible to determine an adjusted modelled IDC which accurately describes the actual time-dependent concentration of the indicator due to a first cycle or passage at the locations. An estimate of the relevant parameters may be derived from the modelled IDC. The invention therefore provides a possibility for modelling and predicting even very low concentrations of indicator present in the bloodstream, with a high degree of accuracy.

Another benefit is that the time interval may be chosen for which the SNR (Signal to Noise Ratio), related to the signal corresponding to the first cycle or passage of the indicator at the location, is large, whereas measurements with a small SNR may be ignored. It will be appreciated that this improves the accuracy of the determined adjusted modelled IDC.

Also, the integral over time of the indicator concentration from the start of the IDC (i.e. the front of the indicator bolus passing the measured location) to infinity (where the concentration related to the first passage of indicator converges to zero) may be calculated. Due to noise, recirculation and other disturbances calculation of this integral directly from the measured signal is impossible. Calculating this integral may be important for determining parameters as volumetric flow and mean transit time and with these parameters, blood volumes.

Further to this, modelling of indicator concentration due to the first passage of thereof allows measurement of an indicator dilution curve due to recirculation (second or further passage of the indicator past the one or more locations). The recirculation concentration time measurements may be achieved by subtraction of the adjusted modelled IDC from the measurements. Analysis of the recirculation concentration time measurements allows measurement of the total blood volume using recirculation mean transit time analysis. The mean transit time due to recirculation provides an indication of the average time that the contrast takes to cover the complete circulatory system.

It has been observed that by measuring, acoustic pressure return signals of an indicator passing the one or more locations using ultrasound imaging, the relation between the acoustic pressure and the indicator concentration for the ultra low concentrations becomes linear.

Further, in another embodiment the invention, wherein the measurements are based on videodensitometry, the descriptive relation is a logarithmic relation.

In another embodiment of the invention, for instance, the relation is scaled by calibrating the relation using reference values based on the imaging technique used.

A person skilled in the art may appreciate that when the characteristics of the relation is known, one may scale the relation based on the measurement values compared to reference values for a given arrangement or equipment used. Alternatively, one may experimentally determine the descriptive relation using calibration techniques, measurements and reference values.

In a further embodiment of the present invention, the ultrasound measurements comprise measurements of harmonic modes of the return signal. In particular, in accordance with yet another embodiment of the invention, the harmonic modes comprise secondary or higher harmonic modes of the return signal.

It has been observed that some harmonic modes above the fundamental mode of the ultrasound signal used are in particular suitable for optimising contrast detection of indicator in the bloodstream. The detection of these harmonic modes in the return signal 1 therefore may allow for the use of even smaller doses of indicator, without loss of accuracy of the results of the measurements.

In another embodiment of the invention, the adjusting over a time interval comprises logarithmic transformation of the indicator concentration time signal.

Since the measurements seem to be well modelled using an exponential function, a logarithmic transformation of the measurement results enables linear modelling of the results. It will be understood that this embodiment reduces the complexity of the modelling and fitting process substantially, and therefore provides a way of speeding up the interpretation of the measured results.

The logarithmic transformation used in this embodiment of the invention provides the possibility of numerous further analysis techniques to be applied here. The accuracy of the acquired results from the measurement may be further enhanced by the analysis techniques that become possible due to the logarithmic transformation. Reducing complexity of the method also provides benefits for the arrangements used for interpreting the results (as it may reduce the required arithmetic capabilities), and in addition to this, it enables non-invasive techniques such as transthoracic measurements since the amount of noise can be reduced while the accuracy can be increased.

Therefore, in another embodiment of the invention, the adjusting over a time interval comprises the use of a linear regression algorithm. For instance, the linear regression algorithm is performed on the logarithmic transformation for providing a least squares estimate of the logarithmic transformation.

The use of a linear regression algorithm becomes possible due to the step of logarithmic transformation and provides a least squares estimate of the logarithmically transformed results. Flow characteristics may be calculated directly from these results, providing a quick and accurate means of determining them. A quick measurement of the physiologic parameters such as cardiac output, ejection fraction and regurgitation fraction facilitates easy diagnosis of many cardiac diseases, and allows fast action in emergency routines.

Accurate determination of the starting time of the IDC (which equals the moment on which the indicator bolus is injected, i.e. the injection time) is important in order to provide an accurate model of the IDC. Additionally also the optimal time interval that is suitable for modelling the IDC (the first time interval) is important. Therefore, in another embodiment of the invention, the first time interval is determined by analysis of the at least one indicator dilution curve (IDC).

Several indicator distribution models may be suitable for modelling the IDC. However, it has been observed that an IDC model based on the Local Density Random Walk (LDRW) model provides accurate results. The LDRW model describes the dispersion of an indicator in a carrier fluid under specific conditions, after an indicator bolus which mathematically takes the form of an Dirac delta function is released in the flow. The model provides an estimate of the indicator concentration over time (as an exponential function) at a certain location after the indicator bolus has travelled over a certain distance. The model is based on Brownian motion theory. Since the Dirac impulse injection is not realistic, deconvolution techniques are used in order to consider a realistic injection. A specific application may use a Wiener-filter deconvolution.

In a preferred embodiment of the invention, the modelling comprises a recursive process, wherein n the recursive process comprises one or more of the steps of adjusting one or more parameters of the modelled indicator dilution curve, performing the logarithmic transformation on the indicator concentration time signal, performing the linear regression algorithm and calculating a mean square error of the modelled indicator dilution curve in relation to the indicator concentration time signal.

In a preferred embodiment of the invention as disclosed above, the recursive process is performed until the mean square error is minimised.

This may be achieved, for instance, by recursively shifting the starting time of the estimated indicator dilution curve (IDC) in time, until the minimum mean square error is reached. Due to the logarithmic transformation and linear regression algorithm that may be used, calculation times of the best fit based on finding the least square error are sufficiently short to enable recursive modelling of the IDC until the best fit is found. This improves the accuracy of the method to a great extend.

Other imaging techniques based on contrast agents which are released in the bloodstream may be used for measuring indicator dilution curves at several locations simultaneously. This may be achieved, for instance, by using videodensitometry on echocardiographic images. This enables easy determination of any mean transit time, such as the recirculation mean transit time and intra-thoracic mean transit time.

Therefore, another embodiment of the invention, wherein the one or more locations comprise the right ventricle of the heart and the left atrium, comprises a step of determining an intra-thoracic mean transit time of the indicator between the right ventricle and the left atrium. This embodiment may be further enhanced by calculating the cardiac output and providing an estimate of the intra-thoracic blood volume.

The above embodiments may be even further enhanced by comparing the intra-thoracic blood volume to the total blood volume estimate for the assessment of the intra-thoracic-to-total-blood-volume ratio.

Another embodiment of the invention comprises a step of determining an ejection fraction from an indicator dilution curve (IDC). This embodiment may be used for comparing the values of the ejection fraction with a reference value, which reference value may be based on geometric estimations of an end-diastolic volume and an end-systolic volume of a ventricle of a heart. This may provide an indication of regurgitation of a mitralic or tricuspid valve of a heart.

Note that, instead of using ultrasound measurements, the method of the invention may be used in combination with any one or more of the following imaging techniques: magnetic resonance imaging (MRI), X-ray based imaging, computed tomography (CT), radio-isotope imaging, positron emission tomography (PET), electrical impedance tomography (EIT), laser based imaging techniques, teraherz imaging, imaging based on spectral analysis and similar imaging techniques.

According to a second aspect of the invention, there is provided an arrangement for determining cardiac parameters of a human or animal body having a central bloodstream comprising an indicator periodically passing one or more locations of the body, the arrangement comprising:

ultrasound means for providing ultrasound measurements of a concentration of the indicator by applying an ultrasound signal to the indicator in the central bloodstream and for measuring a return signal in response to the ultrasound signal, of at least a first cycle of the indicator;

means for determining an indicator concentration time signal from the ultrasound measurements, wherein the means are arranged for determining the indicator concentration time signal from the ultrasound measurements, the indicator being of such a low concentration that there is a descriptive relation between the measurements and the indicator concentration time signal;

means for providing a modelled indicator dilution curve from the first cycle of the indicator concentration time signal and for adjusting the modelled indicator dilution curve over a time interval, wherein the time interval corresponds to at least part of the first cycle leading a further cycle of the indicator; and

analysis means for determining the cardiac parameters from a plurality of modelled indicator dilution curves obtained from a plurality of locations in the central bloodstream.

In a preferred embodiment of the invention, the arrangement according to claim 32, wherein the analysis means are arranged for determining the cardiac parameters from a plurality of modelled indicator dilution curves obtained from ultrasound measurements simultaneously at a plurality of locations in the central bloodstream.

In accordance with another embodiment of the invention, the ultrasound means for providing ultrasound measurements are arranged for providing measurements of the fundamental and harmonic modes, such as secondary or higher harmonic modes, of the return signal.

The arrangement may be further arranged for providing an analysis of a recirculation IDC of the indicator related to a second or further passage of the indicator at the one or more locations.

The arrangement may comprise transesophageal measuring means for taking in vivo ultrasonic measurements of the indicator concentration, which transesophageal measuring means may be arranged for taking measurements from an esophagus of a human or animal body.

It has been observed that measurement of the indicator concentrations passing one or more locations in the heart, such as the left or right ventricle or atrium, provides a higher SNR if performed from the esophagus. However, using harmonic ultrasound imaging techniques, the measurements may also be performed using a transthoracic approach, by using a transthoracic transducer.

An arrangement based on ultrasound measuring techniques may further be either arranged for providing measurements based on videodensitometry (using echocardiographic imaging techniques) or by measuring an acoustic intensity and determining the indicator concentration from the acoustic intensity. Specific embodiments of the invention may be directed to either one of these methods or both.

The arrangement may, instead of comprising the ultrasound means, comprise means for taking the measurements based on another imaging technique used for medical purposes, such as means for performing any one or more of a group comprising magnetic resonance imaging (MRI), X-ray based imaging, computed tomography (CT), radio-isotope imaging, positron emission tomography (PET), electrical impedance tomography (EIT), laser based imaging techniques, teraherz imaging, imaging based on spectral analysis and similar imaging techniques.

The invention also relates to a method for determining an indicator dilution curve of an indicator periodically passing one or more locations of a central bloodstream of a human or animal body, the method comprising the steps of:

applying an ultrasound signal to the indicator in the central bloodstream;

taking ultrasound measurements from fundamental and harmonic modes of a return signal in response to the ultrasound signal, of at least a first cycle of the indicator;

determining an indicator concentration time signal from the ultrasound measurements, the indicator being of such a low concentration that there is a descriptive relation between the measurements and the indicator concentration time signal; and

modelling an indicator dilution curve from the first cycle of the indicator concentration time signal and adjusting the modelled indicator dilution curve over a time interval, wherein the time interval corresponds to at least part of the first cycle leading a further cycle of the indicator.

The invention further relates to an arrangement for determining an indicator dilution curve of an indicator periodically passing one or more locations of a central bloodstream circulation of a human or animal body, the arrangement comprising:

ultrasound means for providing ultrasound measurements of a concentration of the indicator by applying an ultrasound signal to the indicator in the central bloodstream and for measuring fundamental and harmonic modes of a return signal in response to the ultrasound signal, of at least a first cycle of the indicator;

means for determining an indicator concentration time signal from the ultrasound measurements, wherein the means are arranged for determining the indicator concentration time signal from the ultrasound measurements, the indicator being of such a low concentration that there is a descriptive relation between the measurements and the indicator concentration time signal; and

means for providing a modelled indicator dilution curve from the first cycle of the indicator concentration time signal and for adjusting the modelled indicator dilution curve over a time interval, wherein the time interval corresponds to at least part of the first cycle leading a further cycle of the indicator.

It will be understood by the person skilled in the art, that the invention provides a method and arrangement that enables measuring using an ultra low dose of contrast agents present in the bloodstream. The measurements may be carried out for any location along the circulation path of the central bloodstream. Interpretation of these measurements is, in accordance with some embodiments of the invention, based on solid models and may be carried out in numerous non-invasive techniques within the scope of the invention. In a specific embodiment one may correlate low concentrations of contrast with videodensitometry. The modelled indicator-dilution curve predicts physiologic parameters, such as cardiac output, ejection-fraction of both ventricles as well as intrathoracic blood volume, both in transesophageal (TEE) as well as transthoracic (TTE) application.

The invention will be further elucidated by a description of some specific embodiments thereof, which embodiments are directed to ultrasound imaging techniques, with reference to the enclosed drawings. The embodiments described are not meant to limit the invention in any way. The invention is only limited by the scope of the appended claims. It will be appreciated that the invention is likewise applicable to any of the imaging techniques mentioned above.

Note that several well known means of releasing an indicator in a bloodstream are known and may be used. However, releasing the indicator in the bloodstream falls outside the scope of the invention. The invention is generally directed to methods and arrangements for measuring of indicator concentrations in a bloodstream based on an imaging technique, and modelling and interpreting the measurements for determining cardiac parameters.

Brief Description of the Drawings

Figure 1 is schematic view of a human or animal heart and circulatory system.

Figure 2 is a schematic view of an indicator bolus travelling in a carrier fluid within a blood vessel towards a detection plane.

Figure 3 is a schematic drawing of a modelling step of a measuring method according to the invention.

Figure 4 is a graph showing measured indicator concentration values against time of measuring, including a modelled first passage and recirculation IDC.

Figure 5 is a graph of the mean square error of the fitted modelled IDC against the time shift of the curve.

Figure 6 is a schematic drawing of an arrangement according the invention.

Figure 7 shows a calibration graph related to the calibration of an arrangement according to the invention.

Detailed Description of the Drawings

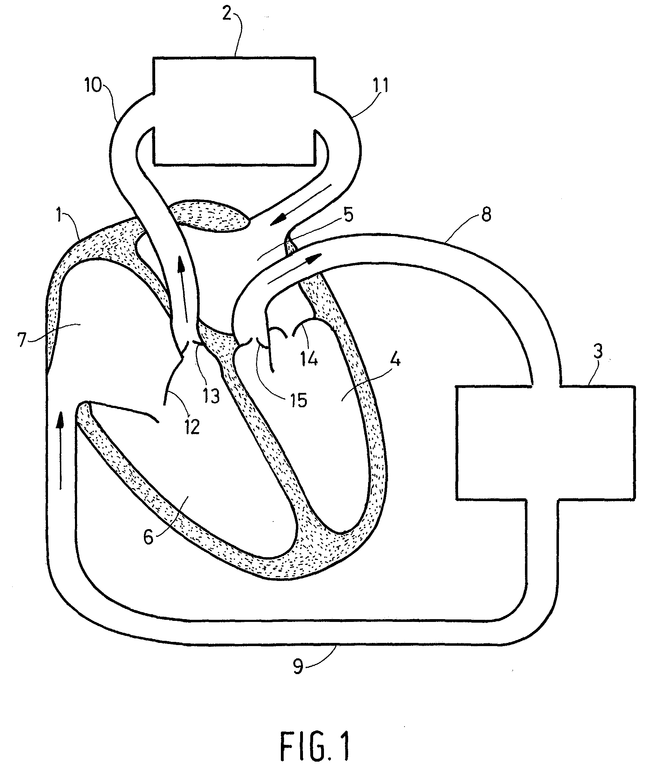

Figure 1 is schematic view of a human or animal heart and circulatory system, showing a heart 1, lungs 2 and the oxygen consuming parts of a body schematically indicated by box 3. The heart 1 pumps blood into the pulmonary circulatory system formed by pulmonary artery 10, lungs 2 and pulmonary veins 11, by contracting the right ventricle 6 on a regular basis. The heart 1 also pumps blood into the rest of the body (systemic circulation) formed by aorta 8, the oxygen consuming parts 3 and (superior and inferior) vena cava 9. From the latter circulation, the (oxygen poor) blood enters the heart again in the right atrium 7. From the pulmonary circulation, the (oxygen rich) blood enters the left atrium 5. Left atrium 5, left ventricle 4 and aorta 8 are separated by mitralic valve 14, aortic valve 15 and right atrium 7, right ventricle 6 and the pulmonary artery 10 are separated by the tricuspid valve 12 and the pulmonary valve 13 respectively.

Due to contractions of the left and right ventricle, the blood is pumped from the heart into circulation. These contractions comprise a diastolic and a systolic phase. In the diastolic phase, the ventricles have large volumes and in systolic phase, the ventricles are contracted and have a smaller volume. Important parameters are the end-diastolic volume ($V_{ed}$) and end-systolic volume ($V_{es}$) of each ventricle, which are the largest volume and the smallest volume of the ventricle respectively.

The cardiac output (C0) is the volumetric flow of blood per unit of time and is a function of the stroke volume (S) (in forward direction in relation to the bloodstream) and the pulse rate (PR) of the heart 1. The stroke volume is the volume of blood that is pumped into circulation each heartbeat, and is (for a healthy person) equal to the difference between $V_{ed}$ and $V_{es}$ of the left ventricle 4.

The ejection fraction (EF) is indicative of the efficiency of a heart and is calculated as follows:

$$EF = \frac{(V_{ed} - V_{es})}{V_{ed}} \cdot 100\%$$

When an indicator bolus is injected directly into the ventricle within a single heart beat, the ejection fraction in forward direction ($EF_f$) may be calculated using the following relation between the indicator concentration at heart beat n ($C_n$) and the indicator concentration at heartbeat n+1 ($C_{n+1}$):

$$EF_f = 1 - \frac{C_{n+1}}{C_n}$$

The intra-thoracic blood volume is the amount of blood in pulmonary circulation and is calculated as the product of the cardiac output (C0) and the intra-thoracic mean transit time (of blood in pulmonary circulation (MTT)).

Regurgitation is a condition of a heart wherein the mitralic valve 14 or tricuspid valve 12 do not sufficiently seal the left or right ventricle respectively. Blood may therefore leak back into the left or right atrium respectively, instead of going into systemic or pulmonary circulation. The regurgitant fraction ($EF_r$) is the difference between the ejection fraction and the ejection fraction in forward direction, i.e. $EF_r = EF - EF_f$.

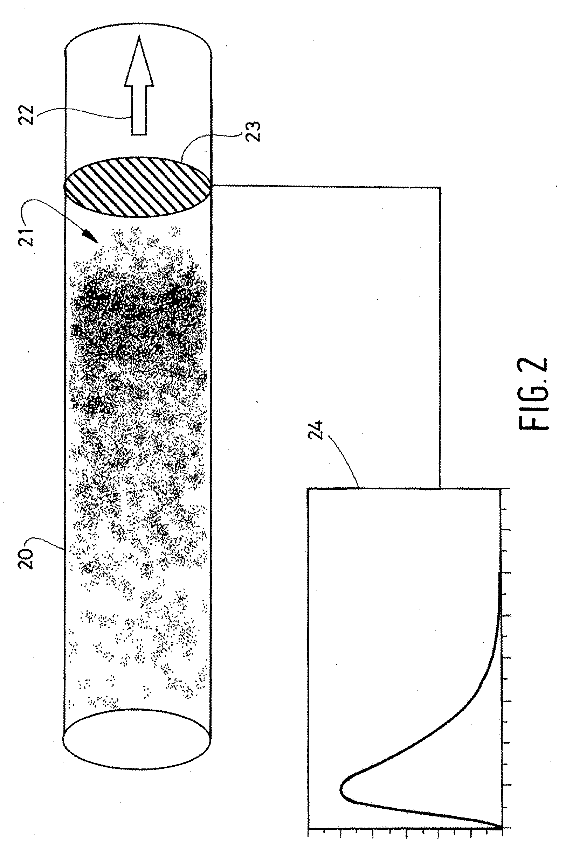

In figure 2, a blood vessel 20 carries a flow of blood (generally indicated by its direction arrow 22) through a body (not shown). An indicator bolus 21 is present in the bloodstream. The indicator may be injected upstream in the vessel. Due to dispersion the indicator bolus is distributed over the vessel (gaussian distribution). Theoretically, if the concentration of the indicator would be measured and plotted against time as it passes the detection plane 23, the result may be an indicator dilution curve (IDC) as indicated by 24 which shows a fast increasing concentration at the front, a maximum indicator concentration, and a long tail where the indicator concentration gradually decreases. In reality however, besides noise that may be present in the measurement results, after a certain period of time, the indicator concentration may rise again showing an upgoing slope on the tail, since the bloodstream is a closed flow system and the indicator is pumped around with the blood.

Recirculation, as described above, makes it difficult to model the first passage of the indicator bolus. In fact, the first passage of indicator is of major importance and the tail of the indicator dilution curve (IDC) comprises most of the information about the physiologic parameters described above. It is therefore important to be able to model all of the indicator dilution curve (IDC) related to the first passage. If an accurate model is found and fitted in order to accurately represent the measured concentration time values, subtraction of this model from the ultrasound measurements provides a recirculation IDC (relating to a second or further passage). This may be achieved according to the invention, by modelling the IDC over a first time interval, for instance where the SNR is sufficiently high, and with the results from the modelling step, provide an estimate of the indicator concentrations in a second time interval lagging the first interval, for instance where the measured concentrations cannot be interpreted due to recirculation or a low SNR (signal-to-noise ratio).

The IDC may be modelled by a model IDC based on Local Density Random Walk (LDRW) statistics. The LDRW statistics is based on Brownian motion of indicator particles within the blood, and provides a model IDC (concentration as a function of time) represented as:

$$C(t) = \frac{m}{\mu q} e^{\lambda} \cdot \sqrt{\frac{\lambda \mu}{2\pi(t - t_0)}} e^{-\frac{\lambda}{2}\left(\frac{(t-t_0)}{\mu} + \frac{\mu}{(t-t_0)}\right)}$$

wherein C(t) is the concentration of the indicator as a function of time t, m is the mass of the injected tracer, q is the volumetric flow of blood per unit of time (indicative of the cardiac output (C0)), $t_0$ is the time on which the indicator is released, $\lambda$ is a parameter related to the diffusion properties of the system, and $\mu$ is the moment ($t - t_0$) on which the centre of mass of the indicator bolus reaches the detection plane after the indicator is released. Note that other models may be used to model the measured results, and the invention is not limited to the use of C(t) as above or the use of a model based on LDRW.

Modelling the measured results in accordance with C(t) is a complex matter due to the non-linear nature of C(t). In order to provide a linear function, enabling linear curve fitting methods, logarithmic transformation of both C(t) and the measured results is necessary. The logarithmic transform of C(t) provides:

$$\ln\left(C\left(t - t_0\right)\right) + \tfrac{1}{2}\ln\left(t - t_0\right) = P_1 - P_2\left(t - t_0\right) - P_3 \frac{1}{\left(t - t_0\right)}$$

$$P_1 = \lambda + \ln\left(\frac{m}{\mu f}\right) + \tfrac{1}{2}\ln\left(\frac{\lambda\mu}{2\pi}\right)$$

$$P_2 = \frac{\lambda}{2\mu}$$

$$P_3 = \frac{\lambda\mu}{2}$$

With $x_1 = (t-t_0)$ and $x_2 = 1/(t-t_0)$, the above is a linear function in $x_1$ and $x_2$ and a linear regression algorithm may be used during modelling of the measured results.

Suppose n measurement samples of indicator concentrations are to be evaluated, each consisting of measurement times $t_{1..n}$ and concentrations $C(t_{1..n}-t_0)$ (note that no measurements before starting time $t_0$ are evaluated). Forming matrix [X] and vector $\underline{Y}$ with the measured values results in

$$[X] = \begin{bmatrix} 1 & x_{11} & x_{21} \\ 1 & x_{12} & x_{22} \\ \vdots & \vdots & \vdots \\ 1 & x_{1n} & x_{2n} \end{bmatrix}$$

and

$$\underline{Y} = \begin{bmatrix} \ln(C(x_{11})) + \tfrac{1}{2}\ln(x_{11}) \\ \ln(C(x_{12})) + \tfrac{1}{2}\ln(x_{12}) \\ \vdots \\ \ln(C(x_{1n})) + \tfrac{1}{2}\ln(x_{1n}) \end{bmatrix}$$

wherein $x_{11}, x_{12} ... x_{1n}$ are equal to $x_1$ as defined above for $t_1, t_2 ... t_n$, and $x_{21}, x_{22} ... x_{2n}$ are equal to $x_2$ as defined above for $t_1, t_2 ... t_n$. The solution of the multiple linear regression of the logarithmic transform of C(t) and the logarithmic transform of the measurements as given by [X] and $\underline{Y}$ is then provided by

$$\underline{P}^{LSE} = \left([X]^t[X]\right)^{-1}[X]^t\,\underline{Y} = \begin{bmatrix} P_1^{LSE} \\ P_2^{LSE} \\ P_3^{LSE} \end{bmatrix}$$

Hence once the vector $\underline{Y}$ and matrix [X] are constructed by logarithmic transformation of the measurements, the vector $\underline{P}^{LSE}$, providing the best fit of C(t) for the measurements may be established by the above equation for $\underline{P}^{LSE}$. Solving the system of three equations in three variables for $P_1$, $P_2$ and $P_3$.

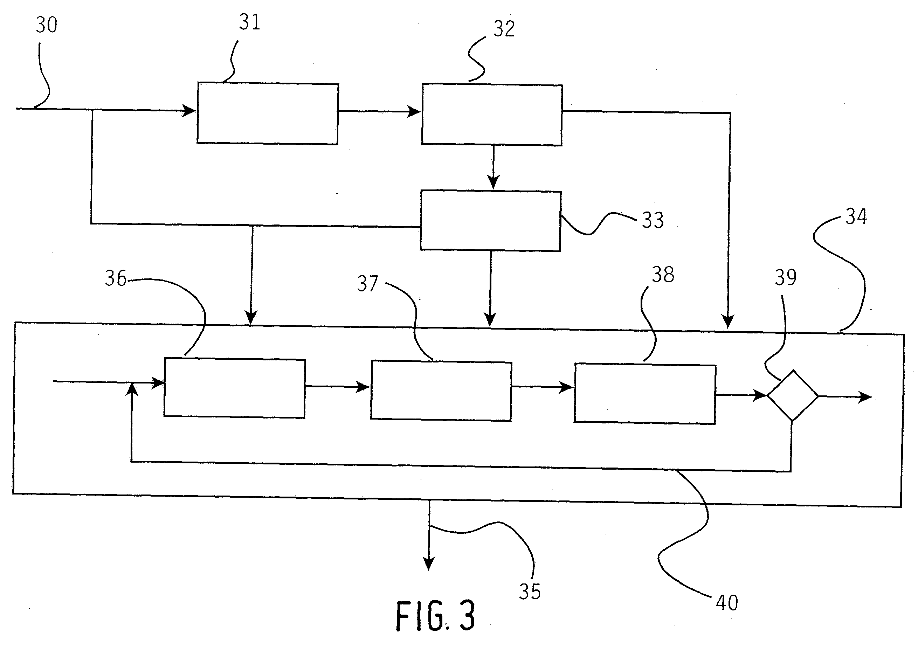

A schematic view of the modelling step is presented in figure 3. Here, the measurements 30, which are first deconvoluted (not shown) in order to compensate for the injection time, are filtered in step 31 using a finite impulse response (FIR) filter to remove high frequency oscillations introduced by the measurement system and some of the other noise introduced in the measurement. The filtered signal is analysed to determine the position of the IDC in the signal and to establish the time interval for performing the linear regression. Note that the end of the time interval may be marked by the appearance of recirculation in the signal and the start of the interval may be marked by the time of the first increase of the indicator concentration. It is especially this last value which is very important to determine and which is often covered by noise.

As a first assumption, the starting time is estimated by a moment in time that is close to but later than the real starting time. One may for instance take the moment on which the concentration first reaches a value which is 10% of the maximum concentration. Similarly, the recirculation time marking the end of the first time interval used for modelling purposes may be estimated the moment on which the concentration reaches 30% of its maximum after the maximum has passed. It will be appreciated that these estimates for the starting time and ending time of the first interval are arbitrarily chosen, for reasons of clarity. Any estimate may be used.

Also, the baseline of the measurements is determined, which is the offset of the concentration. Note that at concentrations equal to the baseline, no indicator is assumed to be present. The baseline is determined in step 33.

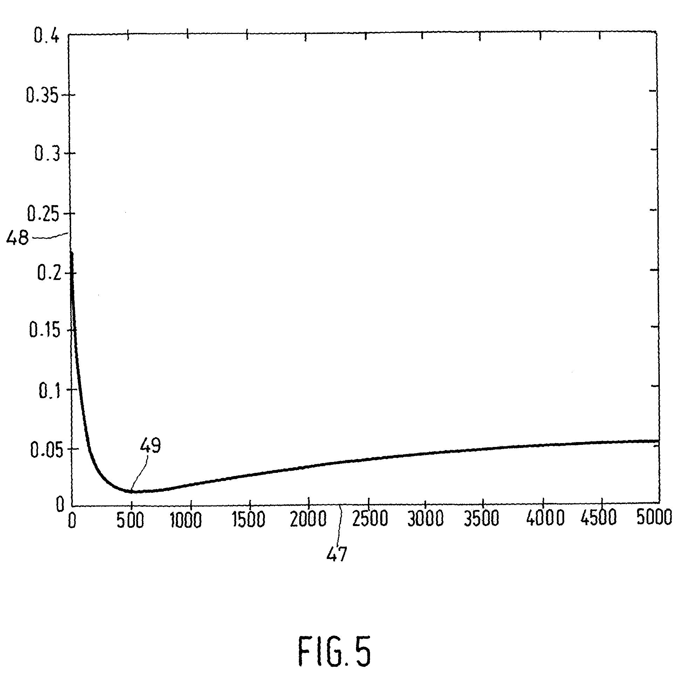

The measured values, the baseline and the determined position of the IDC may then be offered to a recursive process indicated by step 34. The recursive process comprises the recursive steps of shifting the IDC in time (step 36), performing a linear transformation of the measurements using the new starting time $t_0$ (step 37), performing the linear regression step 38 and evaluating the mean square error in step 39. The mean square error has a minimum value when the correct starting time to is used in the logarithmic transformation. This is shown in figure 5, where the mean square error on the vertical axis 48 is plotted against the used time shift in milliseconds on the horizontal axis 47. As can be seen, for a certain value of the time shift (here, about 500 msec) the mean square error shows a minimum value. This minimum value of the mean square error determines the correct value of the time shift for which the logarithmic transformation is performed with the correct starting time $t_0$. In figure 3, the mean square error is evaluated in box 39. When the mean square error has reached a minimum value, the recursion is terminated and the best fit for the given time interval is presented at 35. Otherwise, the process is looped back (step 40) to step 36 (time shift).

Note that not only the time, but also other values may be recursively altered in order to determine the best fit. Note further that due to the logarithmic transformation of the measurements, negative errors in relation to the fitted curve have more influence on the final result than positive errors from the fit. Therefore it is necessary to correct the fitted curve in order to compensate for this effect. The same or a similar procedure may be used in order to provide a recirculation IDC, after the adjusted modelled IDC is subtracted from the ultrasound measurements.

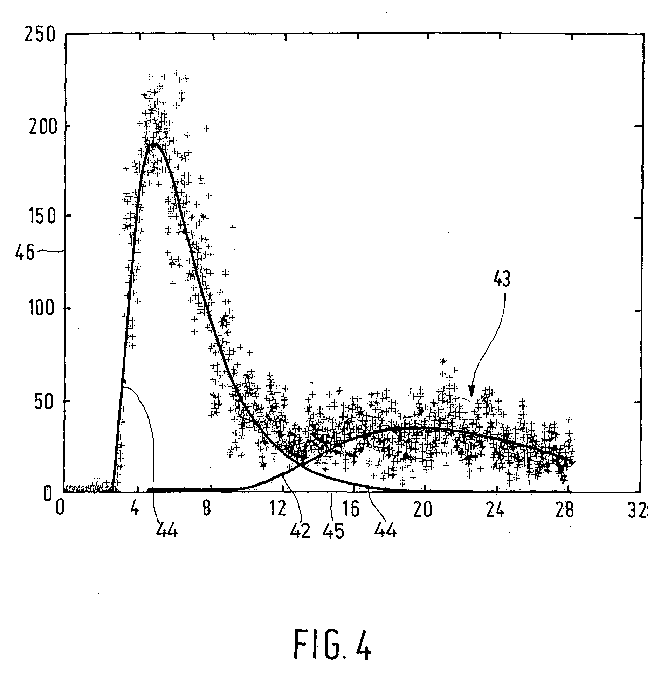

Figure 4 presents a graph wherein on the vertical axis 46, the measured values 43 of the indicator concentration are plotted against time of measurement on the horizontal axis 45, given in seconds. The measurements may be taken by applying an ultrasound signal to one or more locations in the bloodstream, and by measuring a return signal, i.e. the acoustic backscatter intensity signal. One may use the fundamental harmonic or fundamental mode (principal or base frequency) of the return signal and/or the secondary or higher harmonic mode thereof. It has been observed that special harmonic modes of the return signal enhance the measurement of the indicator concentration, as described hereinbefore.

Note that after approximately 12 seconds, the effect of recirculation becomes visible and the measured indicator concentration starts rising again. The best fit related to the first passage of indicator, was modelled for the first part of the measurements before recirculation and is shown as well (indicator dilution curve 44). Note that IDC 44 perfectly matches the first part of the measurements, before recirculation. It may therefore be assumed that IDC 44 in the second part of the graph where recirculation is present, provides an accurate indication of the concentration values in case the effect of recirculation would not be present. The IDC therefore provides a good indication of the indicator concentration versus time related to a first passage of the indicator past a detector or detection plane. A second IDC 42 is modelled using a same method as the first IDC, and is based on the measurements 43 from which the adjusted modelled IDC 44 was subtracted.

Several regions of interest are defined, such that the concentration of ultrasonic agents, i.e. the indicator, may be followed and analysed at different locations at the same time. This enables measurement of any mean transit time between different locations, such as the intra-thoracic mean transit time in case the right ventricle is taken as a first region of interest and the left atrium is taken as a second region of interest.

The volumetric flow and the volumes between different locations are directly derived from the model parameters. In fact, q is already the volumetric flow while the blood volume is determined by the product of q and the mean transit time ($\mu$) of the contrast between different locations. For the total blood volume measurement, the recirculation mean transit time (the difference between first passage and recirculation) is considered. For the intra-thoracic blood volume measurement, the intra-thoracic mean transit time - i.e. the difference between the first passage IDC in the right atrium (or pulmonary artery) and the left atrium - is considered. Instead, the ejection fraction assessment requires in this case extra-calculation. We assume that for t going to infinite, the ventricle refilling is negligible and we may apply the classical $EF_f$ formula as follows:

$$EF_f = \lim_{t \to \infty} 1 - \frac{C_{n+1}}{C_n} = e^{\left(-\lambda t/2\mu\right)}$$

Once the EFf is assessed, if the EF is measured by geometric image analysis thechniques, then the regurgitant ejection fraction is derived as $EF_r = EF - EF_f$.

The method described above is in particular useful in combination with ultrasonic measuring techniques for which a lot of noise is often present in the measurements. These techniques are based on ultrasonic detection of ultrasound contrast agents and often provide low Signal-to-Noise (SNR) ratios which make accurate calculations of physiologic parameters cumbersome. With a low SNR, measuring small concentrations of indicator becomes almost impossible. A person skilled in the art will appreciate that measurement of the tail of the indicator concentration change therefore becomes challenging.

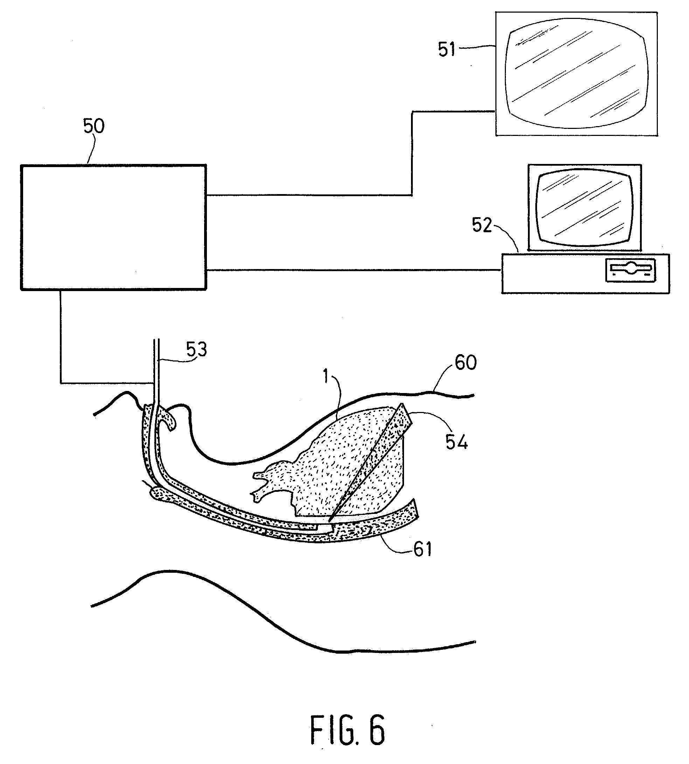

The invention is therefore, according to a second aspect, directed to an ultrasonic measuring arrangement, which is arranged for carrying out the earlier described method. Figure 6 is a schematic view of an arrangement according to the invention. In figure 6, data-acquisition and analysis means 50 are connected to a transducer 53 comprising an ultrasound transducer. Transesophageal transducer 53 may be inserted into the esophagus 61 of a patient 60 in order to provide an echocardiography of his/her heart 1. The transducer 53 in the esophagus 61, defines a detection plane 54. The echocardiography may be visualised on screen 51, on which the ultrasound contrast agents may be visualised as they pass the detection plane 54.

The data-acquisition and analysis means 50 may either be arranged for measuring the acoustic intensity and calculating the indicator concentration based on this intensity, or they may be arranged for videodensitometry of the acquired echocardiographic images. In the latter case, the concentration is derived from the gray levels of the echocardiographic image. It may also be possible to say define a region of interest in the echocardiography. The arrangement may then only evaluate the gray levels in the region of interest.

In accordance with the invention, the data-acquisition and analysis means 50 provide the determination of the indicator delution curves and the calculation of the cardiac parameters therefrom, as extensively discussed above.

The data-acquisition and analysis means 50 may operate using a deconvolution technique, which allows estimating the delution impulse response of the fluid dynamic system between two different locations at which multiple delution curves are measured.

The arrangement may also used in combination with in-vitro measurement of the ultrasound contrast agents. Therefore, transducer 53 (TEE or TTE) may be replaced by a regular transthoracic transducer which is suitable for performing transthoracic measurements.

Note that the results from the data-acquisition and analysis means 50 may also be sent to a personal computer or other computing means 52 for further analysis and/ or visualisation purposes.

Note that the method may also be used with help of a transthoracic transducer (not shown) placed, for instance, on the chest of the patient. The transducer may likewise be connected to the data acquisition and analysis means 50.

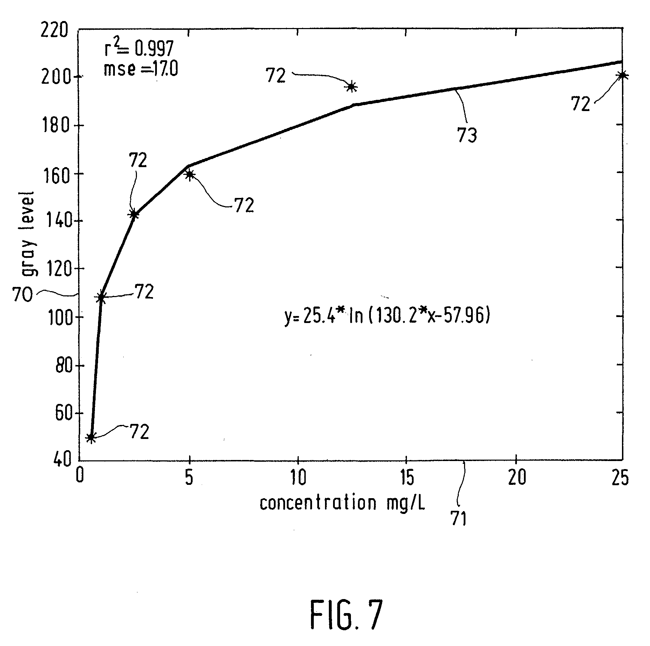

Figure 7 shows a calibration graph related to the calibration of an arrangement according to the invention. The arrangement of this embodiment is based on videodensitometry, wherein a grayscale level in a region of interest is translated into a corresponding concentration value of an indicator. The vertical axis 70 is indicative of the gray level, while the horizontal axis 71 is indicative of the concentration of indicator in the bloodstream in mg/1. Note that the calibrated curve 73 is based on a plurality of measurements 72 for known concentrations. The given descriptive relation (calibrated curve) is a logarithmic relation: y = 25.4 * ln( 130.2 * x - 57.96 ), wherein y corresponds to the gray level and x corresponds to the concentration.

Similarly, for measurements based on acoustic pressure the calibrated curve may be a linear curve.

For purpose of comprehensiveness, it is noted here that numerous modifications and variations of the present invention are possible in the light of the above teachings. It is therefore understood that, within the scope of the appended claims, the invention may be practised otherwise than as specifically described herein.

**Claims**

1.   Method for determining cardiac parameters of a human or animal body having a central bloodstream comprising an indicator periodically passing one or more locations of said body, said method comprising the steps of:

applying an ultrasound signal to said indicator in said central bloodstream;
taking ultrasound measurements from a return signal in response to said ultrasound signal, of at least a first cycle of said indicator;

determining an indicator concentration time signal from said ultrasound measurements, said indicator being of such a low concentration that there is a descriptive relation between said measurements and said indicator concentration time signal;

modelling an indicator dilution curve from said first cycle of said indicator concentration time signal and adjusting said modelled indicator dilution curve over a time interval, wherein said time interval corresponds to at least part of said first cycle leading a further cycle of said indicator;

wherein said cardiac parameters are determined from a plurality of modelled indicator dilution curves obtained from a plurality of locations in said central bloodstream.

2. Method according to claim 1, wherein said cardiac parameters are determined from a plurality of modelled indicator dilution curves obtained from ultrasound measurements simultaneously at a plurality of locations in said central bloodstream.

3. Method according to any of the previous claims, wherein said descriptive relation is a mathematical relation, such as a logarithmic or linear relation.

4. Method according to any of the previous claims, wherein said relation is scaled by calibrating said relation using reference values for said ultrasound measurements.

5. Method according to any of the previous claims, wherein said ultrasound measurements comprise measurements of harmonic modes of said return signal.

6. Method according to claim 5, wherein said harmonic modes comprise secondary or higher harmonic modes of said return signal.

7. Method according to any of the previous claims, wherein said adjusting over said time interval comprises logarithmic transformation of said indicator concentration time signal.

8. Method according to any of the previous claims, wherein said adjusting over said time interval comprises using a linear regression algorithm.

9. Method according to any of the previous claims, wherein said adjusting over said time interval comprises calculating a least squares estimate.

10. Method according to claim 9, wherein said least squares estimate is a linear least squares estimate.

11. Method according to claim 9 or 10 as dependent from claims 7 and 8, wherein said linear regression algorithm is performed on said logarithmic transformation for providing the least squares estimate of the logarithmic transformation.

12. Method according to any of the previous claims, wherein said time interval is determined by analysis of the indicator concentration time signal.

13. Method according to any of the previous claims, further comprising a step of providing an estimate of the indicator concentration over a further time interval lagging said first time interval for at least one of said locations, related to said first cycle.

14. Method according to any of the previous claims, wherein said modelling of an indicator dilution curve is performed using a local density random walk model.

15. Method according to claim 14, wherein said modelled indicator dilution curve comprises the representation:

$$C(t) = \frac{m}{\mu q} e^{\lambda} \cdot \sqrt{\frac{\lambda \mu}{2 \pi \left(t - t_0\right)}} e^{-\frac{\lambda}{2}\left(\frac{\left(t - t_0\right)}{\mu} + \frac{\mu}{\left(t - t_0\right)}\right)}$$

wherein C(t) is proportional to a time-dependent indicator concentration, t is proportional to time, m is proportional to an indicator mass, q is proportional to a volumetric flow of blood, to is proportional to a reference time, λ is proportional to diffusion of said indicator, and μ is proportional to a time at which a centre of mass of said indicator is at said one or more locations.

16. Method according to any of the previous claims, wherein said adjusting over said time interval comprises a recursive process.

17. Method according to claim 16 dependent on claim 7 and claim 8, wherein said recursive process comprises one or more of the steps of adjusting one or more parameters of said modelled indicator dilution curve, performing said logarithmic transformation on said indicator concentration time signal, performing said linear regression algorithm and calculating a mean square error of said modelled indicator dilution curve in relation to said indicator concentration time signal.

18. Method according to claim 17, wherein adjusting one or more parameters comprises shifting said modelled indicator dilution curve in time.

19. Method according to any of the claims 17 and 18, wherein said recursive process is ended after said mean square error is minimised.

20. Method according to claim 13 as dependent on claim 7, wherein after said adjustment, said method comprises a further step of solving a set of equations comprising:

$$P_1 = \lambda + \ln\left(\frac{m}{\mu f}\right) + \tfrac{1}{2}\ln\left(\frac{\lambda\mu}{2\pi}\right)$$

$$P_2 = \frac{\lambda}{2\mu}$$

$$P_3 = \frac{\lambda\mu}{2}$$

wherein estimates of $P_1$, $P_2$ and $P_3$ are based on said adjustment.

21. Method according to any of the previous claims, further comprising a step of determining a volumetric flow of blood of said bloodstream from said modelled indicator dilution curve.

22. Method according to any of the previous claims, wherein said locations comprise one or more chambers of a heart, such as left or right atrium or left or right ventricle.

23. Method according to claim 22, wherein said one or more chambers of the heart at least comprises a right ventricle and a left atrium, further comprising a step of determining an intra-thoracic mean transit time of said indicator between said right ventricle and said left atrium.

24. Method according to claims 21 and 23, comprising a step of determining an intra-thoracic blood volume from said intra-thoracic mean transit time and said volumetric flow of blood.

25. Method according to any of the previous claims, further comprising a step of determining an ejection fraction from at least one indicator dilution curve.

26. Method according to any of the previous claims, comprising a step of subtracting a modelled indicator dilution curve from said ultrasound measurements for determining a recirculation indicator dilution curve corresponding to a further cycle of said indicator passing said locations.

27. Method according to claim 26, further comprising a step of determining a recirculation mean transit time based on said recirculation indicator dilution curve and a modelled indicator dilution curve.

**28.** Method according to claim 27, as dependent on claim 23, further comprising a step of comparing said intra-thoracic mean transit time to said recirculation mean transit time for providing an intra-thoracic-to-total-blood-volume ratio.

**29.** Method according to any of the previous claims, wherein said ultrasound measurements are replaced by an imaging technique comprising any one or more of a group including magnetic resonance imaging (MRI), X-ray based imaging, computed tomography (CT), radio-isotope imaging, positron emission tomography (PET), electrical impedance tomography (EIT), laser based imaging techniques, teraherz imaging, imaging based on spectral analysis and similar imaging techniques.

**30.** Use of a method according to any of the previous claims, said method further comprising a step of determining an ejection fraction from said indicator dilution curve (IDC), wherein said method is used for comparing said values of the ejection fraction with a reference value, which reference value is based on estimations of an end-diastolic volume and an end-systolic volume of a ventricle of a heart.

**31.** Use of a method according to claim 30 for giving an indication of regurgitation of a mitralic or tricuspid valve of a heart.

**32.** Arrangement for determining cardiac parameters of a human or animal body having a central bloodstream comprising an indicator periodically passing one or more locations of said body, said arrangement comprising:

ultrasound means for providing ultrasound measurements of a concentration of said indicator by applying an ultrasound signal to said indicator in said central bloodstream and for measuring a return signal in response to said ultrasound signal, of at least a first cycle of said indicator;
means for determining an indicator concentration time signal from said ultrasound measurements, wherein said means are arranged for determining said indicator concentration time signal from said ultrasound measurements, said indicator being of such a low concentration that there is a descriptive relation between said measurements and said indicator concentration time signal;
means for providing a modelled indicator dilution curve, from said first cycle of said indicator concentration time signal and for adjusting said modelled indicator dilution curve over a time interval, wherein said time interval corresponds to at least part of said first cycle leading a further cycle of said indicator; and
analysis means for determining said cardiac parameters from a plurality of modelled indicator dilution curves obtained from a plurality of locations in said central bloodstream.

**33.** Arrangement according to claim 32, wherein said analysis means are arranged for determining said cardiac parameters from a plurality of modelled indicator dilution curves obtained from ultrasound measurements simultaneously at a plurality of locations in said central bloodstream.

**34.** Arrangement according to claim 32 or 33, wherein said descriptive relation is a mathematical relation, such as a logarithmic or linear relation.

**35.** Arrangement according to any of the claims 32-34, wherein said ultrasound means for providing ultrasound measurements are arranged for providing measurements of harmonic modes of said return signal.

**36.** Arrangement according to claim 35, wherein said harmonic modes comprise secondary or higher harmonic modes of said return signal.

**37.** Arrangement according to any of the claims 32-36, wherein said means for providing ultrasound measurements are arranged for measuring an acoustic intensity and determining said indicator concentration from said acoustic intensity.

**38.** Arrangement according to any of the claims 32-37, further comprising imaging means, and means for determining said indicator concentration time signal from a video signal provided by said imaging means.

**39.** Arrangement according to any of the claims 32-38, wherein said means for providing ultrasound measurements comprise a transducer.

**40.** Arrangement according to any of the claims 32-39, wherein said means for providing ultrasound measurements are arranged for providing in vivo measurements.

**41.** Arrangement according to claim 40, wherein said means for providing ultrasound measurements are further arranged for providing transesophageal measurements.

**42.** Arrangement according to any of the claims 32-41, wherein said means for providing ultrasound measurements are arranged for providing in vitro measurements.

**43.** Arrangement according to claim 42, wherein said means for providing ultrasound measurements are arranged for providing transthoracic measurements.

**44.** Arrangement according to any of the claims 32-43, wherein said ultrasound means are replaced by means for performing an imaging technique comprising any one or more of a group including magnetic resonance imaging (MRI), X-ray based imaging, computed tomography (CT), radio-isotope imaging, positron emission tomography (PET), electrical impedance tomography (EIT), laser based imaging techniques, teraherz imaging, imaging based on spectral analysis and similar imaging techniques.

**45.** Method for determining an indicator dilution curve of an indicator periodically passing one or more locations of a central bloodstream circulation of a human or animal body, said method comprising the steps of:

applying an ultrasound signal to said indicator in said central bloodstream;
taking ultrasound measurements from fundamental and harmonic modes of a return signal in response to said ultrasound signal, of at least a first cycle of said indicator;
determining an indicator concentration time signal from said ultrasound measurements, said indicator being of such a low concentration that there is a descriptive relation between said measurements and said indicator concentration time signal; and
modelling an indicator dilution curve from said first cycle of said indicator concentration time signal and adjusting said modelled indicator dilution curve over a time interval, wherein said time interval corresponds to at least part of said first cycle leading a further cycle of said indicator.

**46.** Arrangement for determining an indicator dilution curve of an indicator periodically passing one or more locations of a central bloodstream circulation of a human or animal body, said arrangement comprising:

ultrasound means for providing ultrasound measurements of a concentration of said indicator by applying an ultrasound signal to said indicator in said central bloodstream and for measuring fundamental and harmonic modes of a return signal in response to said ultrasound signal, of at least a first cycle of said indicator;
means for determining an indicator concentration time signal from said ultrasound measurements, wherein said means are arranged for determining said indicator concentration time signal from said ultrasound measurements, said indicator being of such a low concentration that there is a descriptive relation between said measurements and said indicator concentration time signal; and
means for providing a modelled indicator dilution curve, from said first cycle of said indicator concentration time signal and for adjusting said modelled indicator dilution curve over a time interval, wherein said time interval corresponds to at least part of said first cycle leading a further cycle of said indicator.

FIG. 1

FIG.2

FIG. 3

FIG. 4

FIG.5

FIG. 6

FIG. 7